# EUROPEAN PATENT SPECIFICATION

(11) **EP 2 529 743 B1**
(45) Date of publication and mention of the grant of the patent: **12.08.2015**
(21) Application number: 11737021.3
(22) Date of filing: 26.01.2011
(51) Int. Cl.: A61K 31/80, A61P 1/00

(54) **PROPHYLACTIC OR THERAPEUTIC AGENT FOR CROHN'S DISEASE COMPRISING ORGANIC ACID POLYMER AS ACTIVE INGREDIENT**
PROPHYLAKTIKUM ODER THERAPEUTIKUM GEGEN MORBUS CROHN MIT EINEM ORGANISCHEN SÄUREPOLYMER ALS WIRKSTOFF
AGENT PROPHYLACTIQUE OU THÉRAPEUTIQUE DE LA MALADIE DE CROHN COMPRENANT UN POLYMÈRE D'ACIDE ORGANIQUE COMME PRINCIPE ACTIF

(30) Priority: 28.01.2010 JP 2010016166
(43) Date of publication of application: 05.12.2012
(73) Proprietor: Sanwa Kagaku Kenkyusho Co., Ltd, Nagoya-shi, Aichi 461-8631 (JP)
(72) Inventor: HASHIMOTO Hiroyuki, Nagoya-shi Aichi 461-8631 (JP); KUMAZAWA Toshihiko, Nagoya-shi Aichi 461-8631 (JP); TAKEDA Motohiro, Nagoya-shi Aichi 461-8631 (JP); JOMORI Takahito, Nagoya-shi Aichi 461-8631 (JP); HIBI Chihiro, Nagoya-shi Aichi 461-8631 (JP); MIZUNO Kuniharu, Nagoya-shi Aichi 461-8631 (JP)
(74) Representative: Grünecker Patent- und Rechtsanwälte PartG mbB
(86) International application number: PCT/JP2011/051425
(87) International publication number: WO 2011/093308

(56) References cited:
- EP-A1- 0 652 223
- JP-A- 8 040 916
- JP-A- 8 053 352
- JP-A- 54 115 324
- Hans-Christian Reinecker ET AL: "Monocyte-Chemoattractant Protein 1 Gene Expression in Intestinal Epithelial Cells and Inflammatory Bowel Disease Mucosa", GASTROENTEROLOGY, 1 January 1995 (1995-01-01), pages 40-50, XP055074255, Retrieved from the Internet: URL:http://ac.els-cdn.com/0016508595900063 /1-s2.0-0016508595900063-main.pdf?_tid=e8e 24622-fe7a-11e2-bd12-00000aab0f27&acdnat=1 375781642_bb5da3a0ac1da9f4e6b1b2da96369f48 [retrieved on 2013-08-06]
- SHOJI YOKOCHI ET AL: "An Anti-Inflammatory Drug, Propagermanium, May Target GPI-Anchored Proteins Associated with an MCP-1 Receptor, CCR2", JOURNAL OF INTERFERON & CYTOKINE RESEARCH, vol. 21, no. 6, 1 June 2001 (2001-06-01), pages 389-398, XP055074298, ISSN: 1079-9907, DOI: 10.1089/107999001750277862
- YASUO SUZUKI: 'Crohn-byo Shindan Chiryo no Shin Tenkai' JOURNAL OF JAPAN SOCIETY OF COLOPROCTOLOGY vol. 56, no. 5, 2003, page 216
- YASUSHI IWAO: 'Immunomodulator therapy for maintenance of remission in Crohn's disease' JAPANESE JOURNAL OF GASTROENTEROLOGY vol. 105, no. 5, 2008, pages 659 - 668, XP008163997

## Description

### TECHNICAL FIELD

The present invention relates to a novel pharmaceutical use of an organic acid polymer, specifically of a 3-oxygermylpropionic acid polymer.

### BACKGROUND ART

Crohn's disease (CD) is an intractable disease of unknown cause by which lesions such as stenosis or fistula appear incontinuously on the whole area of the gastrointestinal tract mainly from the oral cavity to the anus. Furthermore, in some cases, the disease is accompanied by extraintestinal complications such as arthritis, iritis, pyoderma gangrenosum and erythema nodosum. In Japan, the disease is designated as one of target diseases (specified diseases) in the Intractable Disease Treatment Research Program, and is an intractable disease that is different from gastric ulcer and duodenal ulcer. There is no radical therapeutic method, and the object of the therapy is to suppress the symptom in the active stage to induce remission, and to maintain the remission state by the medicinal therapy or the like. The bases of an oral pharmacotherapy are 5-aminosalicylic acid (5-ASA) formulations such as mesalazine and salazosulfapyridine, and adrenocortical steroids (prednisolone) in Japan, and in some cases, antibacterial agents (metronidazole), immunosuppressive agents (azathioprine) and the like is administered. On the other hand, adrenocortical steroids and immunosuppressive agents are fundamental in the United States and have been used for treating Crohn's disease (Hans-Christian Reinecker et al.: "Monocyte-Chemoattractant Protein 1 Gene Expression in Intestinal Epithelial Cells and Inflammatory Bowel Disease Mucosa", GASTROENTEROLOGY, 1 January 1995, pages 40-50).

However, no effectiveness in a long term administration aiming at maintenance of remission has been shown for specifically adrenocortical steroids, and there are many problems such as their side effects, steroid dependency and resistance. Furthermore, with respect to mesalazine and salazosulfapyridine that are 5-ASA formulations, indication for Crohn's disease is allowed only in Japan and is not allowed in the United States. It has been actually reported that mesalazine that is a 5-ASA formulation is ineffective for Crohn's disease. Despite of this fact, the formulation is used frequently by off-label use in the United States; under the actual circumstance, the formulation is reluctantly used since there is no safe oral agent. On the other hand, immunosuppressive agents such as azathioprine which have been accepted and used in Japan and the United States show a certain effect; however, side effects such as leukopenia and occurrence of lymphoma have been reported, and thus they cannot be considered to be safe drugs. Therefore, under the present circumstance, there is still no sufficient oral therapeutic drug for Crohn's disease in view of effectiveness and safeness.

Organic acid polymers, specifically 3-oxygermylpropionic acid polymers, have been studied with respect to a number of pharmaceutical use. For example, uses for hypertensive cardiovascular diseases (Jpn. Pat. Appln. Laid-open Publication No. 55-167222), an action of enhancing the production of interferons (Jpn. Pat. Appln. Laid-open Publication No. 2-134818), an action of inhibiting a Maillard reaction (Jpn. Pat. Appln. Laid-open Publication No. 8-059485), an action of enhancing the production of IL-10 (Jpn. Pat. Appln. Laid-open Publication No. 11-049683), uses for arteriosclerotic diseases (Jpn. Pat. Appln. Laid-open Publication No. 2000-229856), an action of antagonizing MCP-1 (Jpn. Pat. Appln. Laid-open Publication No. 2000-136139), use for Type II diabetic nephropathy (Jpn. Pat. Appln. Laid-open Publication No. 2003-81843) and the like have been reported.

Many supposed pharmaceutical uses of organic acid polymers are listed in the prior art documents, and as one of them, an action of healing and repairing ulcers in the gastrointestinal system, an action of controlling the function of the large intestine (Jpn. Pat. Appln. Laid-open Publication No. 54-115324, Jpn. Pat. Appln. Laid-open Publication No. 54-116100), uses for ulcers in the stomach, duodenum and large intestine (Jpn. Pat. Appln. Laid-open Publication No. 52-51327) and the like are described. As mentioned above, many pharmaceutical uses have been reported on organic acid polymers, but use for Crohn's disease has not been reported.

Patent Literature 1: Jpn. Pat. Appln. Laid-open Publication No. 55-167222
Patent Literature 2: Jpn. Pat. Appln. Laid-open Publication No. 2-134818
Patent Literature 3: Jpn. Pat. Appln. Laid-open Publication No. 8-059485
Patent Literature 4: Jpn. Pat. Appln. Laid-open Publication No. 11-049683
Patent Literature 5: Jpn. Pat. Appln. Laid-open Publication No. 2000-229856
Patent Literature 6: Jpn. Pat. Appln. Laid-open Publication No. 2003-81843
Patent Literature 7: Jpn. Pat. Appln. Laid-open Publication No. 2000-136139
Patent Literature 8: Jpn. Pat. Appln. Laid-open Publication No. 54-115324
Patent Literature 9: Jpn. Pat. Appln. Laid-open Publication No. 54-116100
Patent Literature 10: Jpn. Pat. Appln. Laid-open Publication No. 52-51327

### DISCLOSURE OF THE PRESENT INVENTION

### Problem to be Solved by the Invention

The present invention aims at providing an agent for use in treating Crohn's disease.

### Means for Solving the Problem

The present inventors have evaluated the medicinal effect and pharmacology of an organic acid polymer, specifically a 3-oxygermylpropionic acid polymer, in a mouse 2,4,6-trinitrobenzenesulfonic acid (TNBS)-induced Crohn's disease model. A mouse TNBS-induced Crohn's disease model is a general experimental animal model that is said to be similar to human Crohn's disease. As the result thereof, they have found that the compound has significant medicinal and pharmacological effects, and completed the present invention.

Namely, the present invention is an agent for use in preventing or treating Crohn's disease, comprising an organic acid polymer represented by the following formula:

[(O_{1/2})₃Ge-A-CO₂H]ₙ

wherein n is 100-1,000, and A is a C-C3 lower alkyl group.

As such organic acid polymer, a 3-oxygermylpropionic acid polymer with an ethylene group for A is preferable.

In the 3-oxygermylpropionic acid polymer, the degree of polymerization n is preferably 200-900. Such 3-oxygermylpropionic acid polymer can be specified by the following physical properties. Namely, the 3-oxygermylpropionic acid polymer that has the following features in powder X-ray diffraction spectrum: a large diffraction peak around 6.5° and relatively large diffraction peaks around 11.6°, 13.8°, 18.4°, 21.2°, and 22.4°, respectively. The 3-oxygermylpropionic acid polymer that has the following features of absorption in IR spectrum: large absorption bands around 800 cm⁻¹, 900 cm⁻¹, and 1,700 cm⁻¹, respectively, and relatively large absorption bands around 560 cm⁻¹, 705 cm⁻¹, 760 cm⁻¹, 780 cm⁻¹, 1,250 cm⁻¹, 1,350 cm⁻¹, and 1,400 cm⁻¹, respectively, the absorption band around 1,400 cm⁻¹ being a doublet. The 3-oxygermylpropionic acid polymer that has the following features of chart in DSC: a peak initiation point around 237°C, a peak top around 256°C, a peak end point around 276°C and an amount of heat ΔH of approximately 59 mcal/mg. The 3-oxygermylpropionic acid polymer that has a molecular formula: (C₃H₅GeO_{3.5})ₙ, a weight average degree of polymerization: n = 548 ± 337, and a weight average molecular weight: average value ± standard error = 9.29 × 10⁴ ± 5.72 × 10⁴.

### Advantageous Effects of the Invention

The organic acid polymer of the present invention is effective as an agent for use in treating Crohn's disease.

### BRIEF DESCRIPTION OF DRAWINGS

FIG. 1 shows a powder X-ray diffraction spectrum of propagermanium.
FIG. 2 shows an IR spectrum of propagermanium.
FIG. 3 shows DSC of propagermanium.

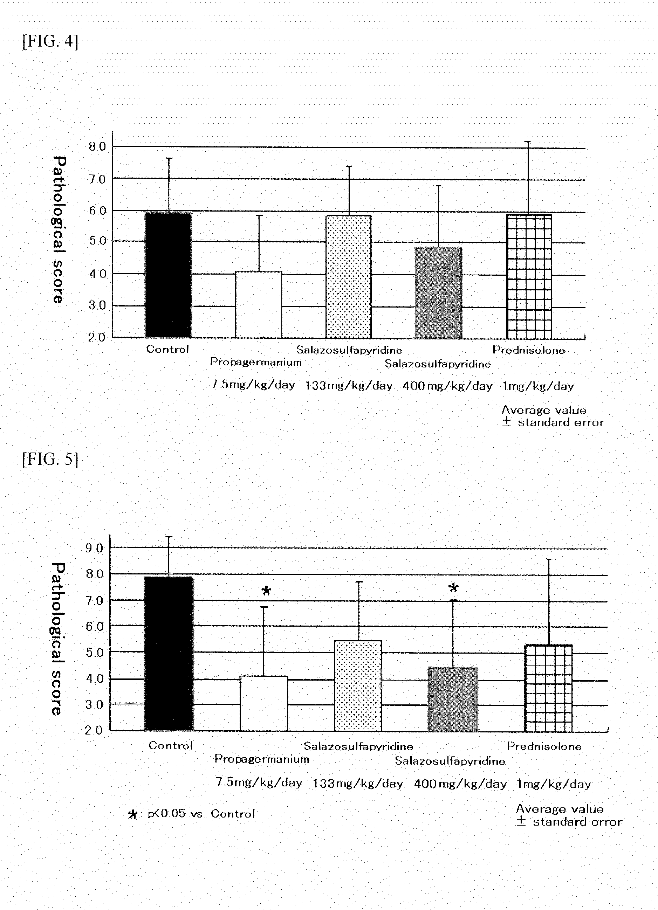

FIG. 4 shows pathological scores by an administration of propagermanium, salazosulfapyridine and prednisolone in mouse TNBS-induced Crohn's disease models (the effects on the first day of TNBS-initiation).
FIG. 5 shows pathological scores by the administration of propagermanium, salazosulfapyridine and prednisolone in mouse TNBS-induced Crohn's disease models (the effects on the second day of TNBS-initiation).

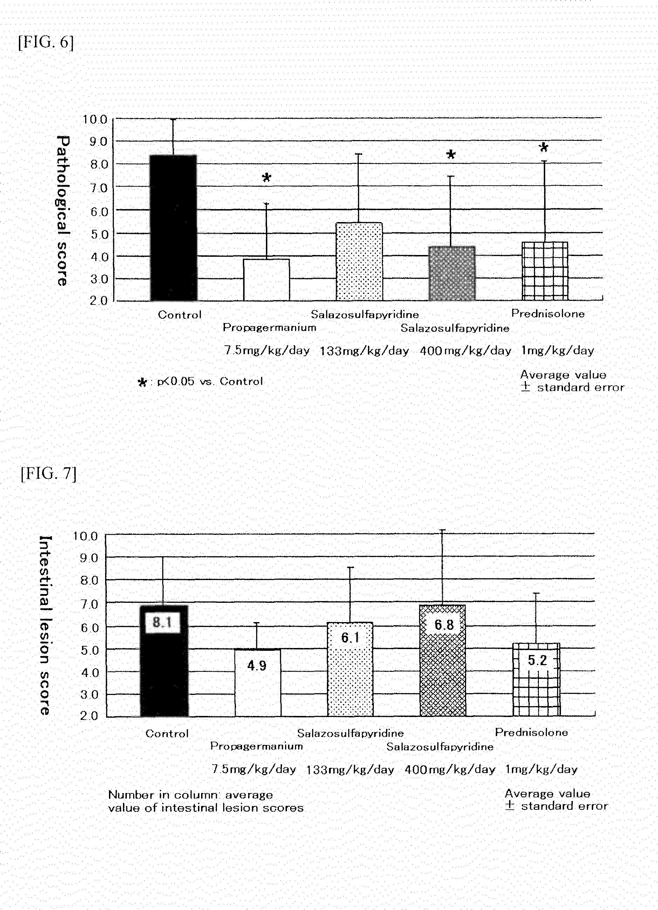

FIG. 6 shows pathological scores by the administration of propagermanium, salazosulfapyridine and prednisolone in mouse TNBS-induced Crohn's disease models (the effects on the third day of TNBS-initiation).
FIG. 7 shows intestinal lesion scores by the administration of propagermanium, salazosulfapyridine and prednisolone in mouse TNBS-induced Crohn's disease models.

### BEST MODE FOR CARRYING OUT THE INVENTION

The organic acid polymer used in the present invention is a known compound, and is represented by the following chemical formula:

[(O_{1/2})₃Ge-A-CO₂H]ₙ

wherein n is 100-1,000, and A is a C1-C3 lower alkyl group.

Specifically, a 3-oxygermylpropionic acid polymer with an ethylene group for A is preferable. In the 3-oxygermylpropionic acid polymer, propagermanium having a degree of polymerization n of 200-900 is well known. The steric structure thereof is presumed to be an 8-membered ring structure represented by the following formula:

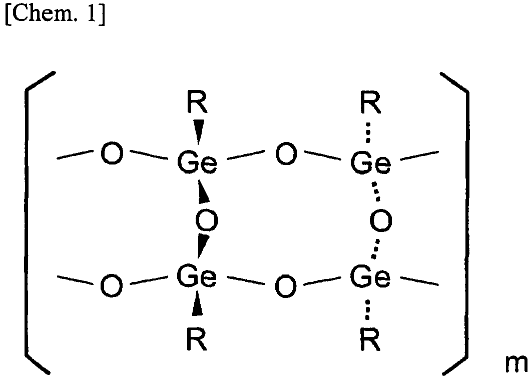

Wherein R represents -CH₂CH₂COOH, and m is a weight average degree of polymerization obtained by converting from the weight average molecular weight of a propagermanium propyl ester and is 137 ± 84 (average value ± standard error 3σ). The minimum structural unit: (O_{1/2})₃GeCH₂CH₂COOH, experimental formula: C₆H₁₀Ge₂O₇.

The propagermanium can be produced according to the method described in Jpn. Pat. Appln. Laid-open Publication No. 2003-81843 or the like. Furthermore, the literature and the like describe that the propagermanium has the physical properties shown in the following Tables 1 and 2. Table 1 shows the result of the molecular weight measurement by a light scattering method, and Table 2 shows a lattice constant obtained by a powder X-ray analysis.

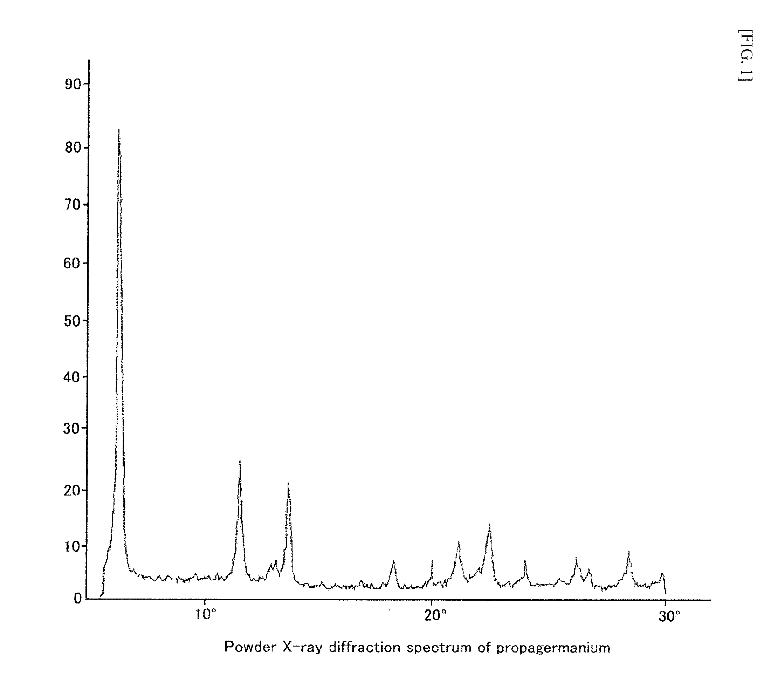

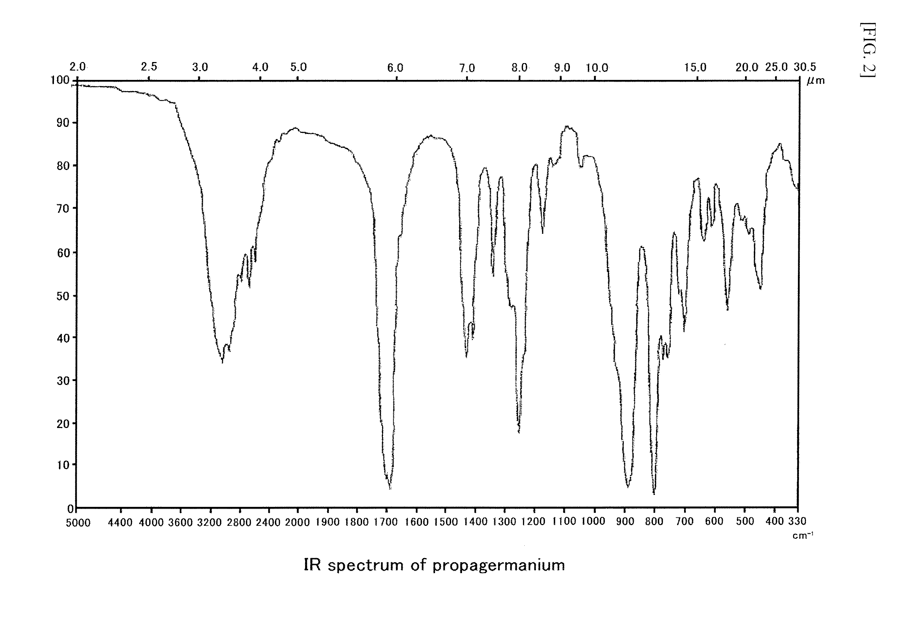

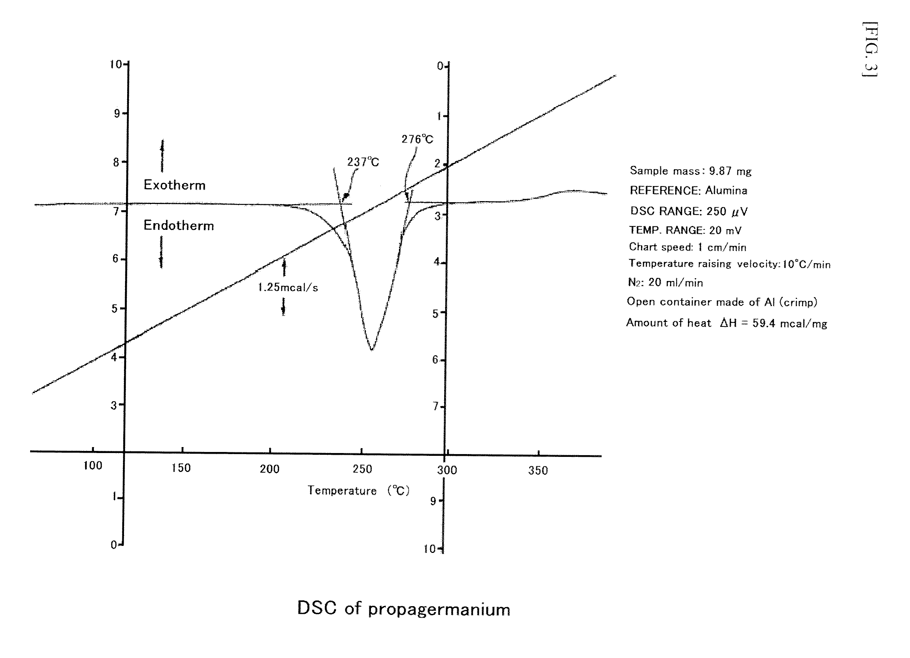

The propagermanium can be specified as a compound having the following physical properties according to FIGS. 1 to 3 (cited from Jpn. Pat. Appln. Laid-open Publication No. 54-115324). It has the following features in powder X-ray diffraction spectrum: a large diffraction peak around 6.5° and relatively large diffraction peaks around 11.6°, 13.8°, 18.4°, 21.2°, and 22.4°, respectively. It has the following features of absorption in IR spectrum: large absorption bands around 800 cm⁻¹, 900 cm⁻¹, and 1,700 cm⁻¹, respectively, and relatively large absorption bands around 560 cm⁻¹, 705 cm⁻¹, 760 cm⁻¹, 780 cm⁻¹, 1,250 cm⁻¹, 1,350 cm⁻¹, and 1,400 cm⁻¹, respectively, the absorption band around 1,400 cm⁻¹ being a doublet. It has the following features of chart in DSC: a peak initiation point around 237°C, a peak top around 256°C, a peak end point around 276°C and an amount of heat ΔH of approximately 59 mcal/mg.

**[Table 1]**

| Weight average molecular weight of propagermanium | | |
|---|---|---|
| | Propagermanium propyl ester | Propagermanium (corresponding value) |
| Weight average molecular weight (Mw) | | |
| Average value (X̅) | 1.16 x 10⁵ | 9.29 x 10⁴ |
| Standard error (3s) | ± 0.71 x 10⁵ | ± 5.72 x 10⁴ |
| Molecular formula* | (C₆H₁₁GeO_{3.5})ₙ | (C₃H₅GeO_{3.5})ₙ |

| Weight average degree of polymerization (n)* | | |
|---|---|---|
| | 548 ± 337 | 548 ± 337 |

| | | |
|---|---|---|
| * is a value in the case the minimum structural unit of propagermanium is (O_{1/2})₃GeCH₂CH₂COOH. | | |

**[Table 2]**

| Lattice constant of propagermanium | |
|---|---|
| Chemical formula ^{*1} | (C₃H₅GeO_{3.5})ₙ |
| Formula weight ^{*1} | 169.66 |
| Crystal class | monoclinic |
| Space group | ---- |
| Unit cell parameters | |
| a (Å) | 13.35 ^{*1} |
| b (Å) | 5.03 ^{*1} |
| c (Å) | 7.55 ^{*1} |
| *β* (deg.) | 94.3 ^{*1} |
| vol (Å³) | 505.4 ^{*2} |
| z | 4 ^{*3} |
| density (gcm⁻³) | 2.23 ^{*4} |

| | |
|---|---|
| *1 The repeating unit of propagermanium is represented as (O_{1/2})₃GeCH₂CH₂COOH. *2 Calculated from the lattice constant *3 Calculated from the lattice constant and measured density *4 Measured by a flotation method | |

In the case the 3-oxygermylpropionic acid polymer, particularly the propagermanium used in the present invention is actually administered to a human, it is preferably used as a composition that is prepared so as to contain excipients in the range of 0.005 part by mass to 50 parts by mass with respect to the compound in the range of 0.005 part by mass to 5 parts by mass. As the excipients, sugars such as lactose, sucrose and dextrans, cellulose-based polymeric substances such as hydroxypropyl cellulose, and natural polymer substances such as albumin are used. Furthermore, although the compound is generally used as an oral formulation, it can also be utilized as a suppository, a nasal cavity formulation, an injection formulation or the like. The dose in the case the compound is administered to a human is within the range of 1 mg to 1,500 mg per day depending on the dosage form, the age of the patient, and the like, and is preferable within the range of 10 mg to 120 mg per day for oral administration to an adult human having a body weight of 50 kg. Furthermore, the formulation of the compound can be conducted according to the description of the formulation example of Jpn. Pat. Appln. Laid-open Publication No. 2003-81843 or the like.

Crohn's disease is an idiopathic chronic disease of unknown cause by which nonspecific lesions appear incontinuously on the whole area of the gastrointestinal tract mainly from the oral cavity to the anus.

The clinical symptoms thereof are abdominal pain, general malaise, diarrhea, bloody stool, fever, weight-loss, anemia, ileus, abdominal mass, nausea, vomiting, peritonitis, and the like. Crohn's disease simultaneously causes nutritional disorder, as well as various gastrointestinal and extraintestinal symptoms, for example, severe symptoms such as intestinal stenosis, ileus, internal fistula, external fistula, intestinal perforation, abdominal mass and massive bleeding. Furthermore, in some cases, the disease is accompanied by extraintestinal complications such as arthritis, iritis, pyoderma gangrenosum and erythema nodosum.

As the treatment method therefore, a nutrition therapy or medicinal therapy is mainly adopted, but a radical operation therapy cannot be expected.

### Examples

The preparation example of the substance of the present invention will be shown below.

### Preparation Example 1

Germanium dioxide and 50% hypophosphorous acid (1.1-fold mol) were reacted in the presence of concentrated hydrochloric acid (5.0-fold mol) at 60 to 80°C for 4 hours, concentrated hydrochloric acid (5.0-fold mol) was added thereto, and acrylic acid (1.1-fold mol) was added dropwise thereto at 40°C or less. The precipitated crystal was collected by filtration and washed with concentrated hydrochloric acid to obtain 3-trichlorogermylpropionic acid (yield 98%). The obtained 3-trichlorogermylpropionic acid was then dissolved in acetone (17-fold mol) and filtered, thereafter water (70-fold mol) was added dropwise thereto at 0°C under stirring, and the mixture was further stirred for 6 hours and stood still for 16 hours. The precipitated crystal was collected by filtration and washed with acetone to obtain a 3-oxygermylpropionic acid polymer (yield 92%).

The obtained 3-oxygermylpropionic acid polymer, i.e., propagermanium, was further induced into a propyl ester form, and thereafter the molecular weight was measured by a light scattering method, and the lattice constant was measured by a powder X-ray analysis method. The results were as shown in Table 1 and Table 2, respectively.

Next, the example of the pharmacological test on the medicinal effect of the propagermanium will be shown. In the pharmacological test on the medicinal effect, the propagermanium was evaluated in a mouse TNBS-induced Crohn's disease model.

Since weight-loss, diarrheal bloody stool and intestinal lesions occur in the model, the model is a general pathological animal model that is said to be similar to human Crohn's disease.

As two kinds of positive control agents, prednisolone and salazosulfapyridine were used. Prednisolone is one of major drugs for the treatment of Crohn's disease as an adrenocortical steroid drug. Since salazosulfapyridine is a prodrug form of a 5-ASA formulation and is converted to 5-ASA in the large intestine, it is a more preferable positive control drug than 5-ASA formulations in view of the drug concentration in the intestinal tract that is a site of action in animal tests.

In this pharmacological test on the medicinal effect, the propagermanium produced in the above-mentioned Preparation Example 1 was used.

### Example 1 of Pharmacological Test on Medicinal Effect

### 1. Experimental method

Seven-week-old male BALB/c Cr Slc mice were purchased from Japan SLC, Inc. After 5 days of breeding for taming, the mice were divided into 5 groups so as to eliminate the difference between body weights, and subjected to an experiment.

The hair on 2 square-cm of the abdomen of the mouse was shaved, and 1% TNBS was applied to the skin. At 7 days after the application of TNBS, the mouse was fixed under anesthesia by halothane inhalation, and 100 µL of 2.0% TNBS was injected from the rectum to initiate intestinal lesions in Crohn's disease.

In the propagermanium-administered group, 0.015% of the propagermanium was mixed with a standard feedstuff for mice CRF-1 (manufactured by Oriental Yeast Co., Ltd.) and administered by ad libitum feeding from immediately after the injection of TNBS to 3 days after the initiation. The average dose during the period of the administration of the propagermanium was calculated to be about 7.5 mg/kg/day as a dose of the propagermanium per day, from the body weight and food consumption.

In the prednisolone-administered group as a positive control, prednisolone was administered once a day as 10 mL/kg of 0.5% CMC suspension using an oral canula for mice from immediately after the injection of TNBS to 3 days after the initiation. The dose was set to be 1 mg/kg/day that is the maximum amount of a clinical general dose (30 to 40 mg/day/person).

In the salazosulfapyridine-administered group as a positive control, salazosulfapyridine was administered twice a day for 3 days as 10 mL/kg of 0.5% CMC suspension using an oral canula for mice from immediately after the injection of TNBS to 3 days after the initiation. Two dose groups: a low dose group (133 mg/kg/day) that is the maximum amount in view of a clinical general dose and a high dose group (400 mg/kg/day) that is effective in rat TNBS models, were set.

### 2. Evaluation items

### (1) Pathological score

At 1, 2 and 3 days after the TNBS initiation, the pathological score (the total of the scores of weight-loss, diarrhea/loose stool and occult blood/hemorrhage) was evaluated according to the following criteria.

### (A) Weight-loss score (when the body weight before the TNBS initiation is considered to be 100%)

0: No decrease
1: Decreased by within 5%
2: Decreased by 5-10%
3: Decreased by 10-20%
4: Decreased by more than 20%

### (B) Diarrhea/loose stool score

0: Normal. A solid form is maintained.
2: Semi-solid loose stool. Wet, and readily collapses when being touched.
4: Diarrhea, with flowability.

### (C) Occult blood/hemorrhage score

0: Normal. No blood is incorporated in stool.
2: Occult blood. Blood is incorporated in stool. Perianal is slightly dirty.
4: Bloody stool. Blood is incorporated in stool. Perianal is dirty with blood.

### (2) Intestinal lesion score

At 3 days after the TNBS initiation, Evans Blue was administered intravenously. At 30 minutes after the administration, the mouse was sacrificed by cervical dislocation, from the colon to the anus were excised and the inner cavity was washed with physiological saline, the lumen was opened, and the degree of the lesions was scored. The intestinal lesion score was evaluated by observing the colon by using a stereomicroscope according to the following criteria.
0: No damage.
1: Hyperemia
2: Hyperemia and thickening of gut walls. No ulcer.
3: Ulcer is present, but is not thickened.
4: Plural lesion sites are present.
5: Widespread lesion sites (1 cm or more in total)
6: Widespread lesion sites (1.5 cm or more in total)
7: Widespread lesion sites (2 cm or more in total)
8: Widespread lesion sites (2.5 cm or more in total)
9: Widespread lesion sites (3 cm or more in total)
10: Widespread lesion sites (3.5 cm or more in total)

### 3. Results and Discussion

### (1) Effect on pathological score

In the 7.5 mg/kg of propagermanium-administered group, a significant effect of improving the pathological score was confirmed at 2 days and 3 days after the initiation. On the other hand, a significant improving effect was not observed in the 133 mg/kg of salazosulfapyridine-administered group. In the 400 mg/kg of salazosulfapyridine-administered group, an improving effect was found at 2 days and 3 days after the initiation, but the effect was weaker than that of the propagermanium group.

On the other hand, in the group of 1 mg/kg of prednisolone, whose reliability is the highest in view of an effect of inducing remission against Crohn's disease, was administered, the effect was not observed at 2 days after the initiation and the effect was observed only at 3 days after the initiation, but the improving effect was weaker than that of the 7.5 mg/kg of the propagermanium-administered group.

### (2) Intestinal lesion score

The effect of improving the intestinal lesion score was the most excellent in the 7.5 mg/kg of propagermanium-administered group, as compared to the 133 mg/kg of salazosulfapyridine-administered group, the 400 mg/kg of salazosulfapyridine-administered group and the 1 mg/kg of prednisolone-administered group.

Namely, as compared to the control group (score 8.1) on lesion, the score was decreased to 4.9 in the propagermanium group; the decreasing effect was weak as 6.1 in the salazosulfapyridine 133 mg/kg group and 6.8 in the salazosulfapyridine 400 mg/kg group; the decreasing effect was 5.2 in the prednisolone 1 mg/kg group; and thus the score was the most excellent in the propagermanium group.

From these results, since the propagermanium showed a more excellent effect than those of prednisolone and salazosulfapyridine, effects on both induction of remission and maintenance of remission state in Crohn's disease can be expected.

## Claims

1. An agent for use in the prevention or the treatment of Crohn's disease, comprising an organic acid polymer represented by the following formula:
[(O1/2)3Ge-A-CO2H]ₙ
wherein n is 100-1,000, and A is a C1-C3 lower alkyl group.

2. The agent for use according to claim 1, wherein the organic acid polymer is a 3-oxygermylpropionic acid polymer with an ethylene group for A.

3. The agent for use according to claim 2, wherein the degree of polymerization n of the 3-oxygermylpropionic acid polymer is 200-900.

4. The agent for use according to claim 2, wherein the 3-oxygermylpropionic acid polymer has the following features in powder X-ray diffraction spectrum:
a large diffraction peak: around 6.5°,
relatively large diffraction peaks: around 11.6°, 13.8°, 18.4°, 21.2°, and 22.4°, respectively.

5. The agent for use according to claim 2, wherein the 3-oxygermylpropionic acid polymer has the following features of absorption in IR spectrum:
large absorption bands: around 800 cm⁻¹, 900 cm⁻¹, and 1,700 cm⁻¹, respectively,
relatively large absorption bands: around 560 cm⁻¹, 705 cm⁻¹, 760 cm⁻¹, 780 cm⁻¹, 1,250 cm⁻¹, 1,350 cm⁻¹, and 1,400 cm⁻¹, respectively, the absorption band around 1,400 cm⁻¹ being a doublet.

6. The agent for use according to claim 2, wherein the 3-oxygermylpropionic acid polymer has the following features of chart in DSC:
peak initiation point: around 237°C,
peak top: around 256°C,
peak end point: around 276°C,
amount of heat ΔH = approximately 59 mcal/mg.

7. The agent for use according to claim 2, wherein the 3-oxygermylpropionic acid polymer is a compound that has:
a molecular formula: (C₃H₅GeO_{3.5})ₙ,
a weight average degree of polymerization: n = 548 ± 337, and
a weight average molecular weight: average value ± standard error = 9.29 x 10⁴ ± 5.72 x 10⁴.

## Patentansprüche

1. Stoff zur Verwendung in der Prävention oder Behandlung von Morbus Crohn, umfassend ein Polymer einer organischen Säure der folgenden Formel:
[(O1/2)3Ge-A-CO2H]ₙ
wobei n 100-1000 ist und A eine C1-C3 Niederalkylgruppe.

2. Stoff zur Verwendung gemäß Anspruch 1, wobei das Polymer der organischen Säure eine 3-Oxygermylpropionsäure mit A als Ethenylgruppe ist.

3. Stoff zur Verwendung gemäß Anspruch 2, wobei der Grad der Polymerisation n der 3-Oxygermylpropionsäure 200-900 ist.

4. Stoff zur Verwendung gemäß Anspruch 2, wobei das Polymer der 3-Oxygermylpropionsäure die folgenden Merkmale in einem Röntgenbeugungsspektrum hat:
großes Maximum: etwa 6,5°
relativ große Maxima: jeweils bei etwa 11,6°, 13,8°, 18,4°, 21,2° und 22,4°.

5. Stoff zur Verwendung gemäß Anspruch 2, wobei das Polymer der 3-Oxygermylpropionsäure die folgenden Absorptionsmerkmale in einem IR Spektrum hat:
große Absorptionsbanden: bei jeweils etwa 800 cm⁻¹, 900 cm⁻¹ und 1700 cm⁻¹,
relativ große Absorptionsbanden: bei jeweils etwa 560 cm⁻¹, 705 cm⁻¹, 760 cm⁻¹, 780 cm⁻¹, 1250 cm⁻¹, 1350 cm⁻¹ und 1400 cm⁻¹, wobei die Absorptionsbande bei 1400 cm⁻¹ ein Duplet ist.

6. Stoff zur Verwendung gemäß Anspruch 2, wobei das Polymer der 3-Oxygermylpropionsäure die folgenden Diagrammmerkmale in DSC hat:
Peakanfangspunkt: etwa 237°C
Peakmaximum: etwa 256°C
Peakendpunkt: etwa 276°C
Wärmemenge ΔH = ungefähr 59 mcal/mg.

7. Stoff zur Verwendung gemäß Anspruch 2, wobei das Polymer der 3-Oxygermylpropionsäure ein Stoff ist der:
eine Summenformel: (C₃H₅GeO_{3,5})ₙ,
einen gewichteten mittleren Grad der Polymerisation: n = 548 ± 337, und
ein gewichtetes mittleres Molekulargewicht: Durchschnitt ± Standardfehler = 9,29 x 10⁴ ± 5,72 x 10⁴
hat.

## Revendications

1. Agent pour utilisation dans la prévention ou le traitement de la maladie de Crohn, comprenant un polymère d'acide organique représenté par la formule suivante :
[(O1/2)3Ge-A-CO2H]ₙ
dans laquelle n vaut 100 à 1000, et A est un groupe alkyle inférieur en C1-C3.

2. Agent pour utilisation selon la revendication 1, dans lequel le polymère d'acide organique est un polymère d'acide 3-oxygermylpropionique avec un groupe éthylène pour A.

3. Agent pour utilisation selon la revendication 2, dans lequel le degré de polymérisation n du polymère d'acide 3-oxygermylpropionique vaut 200 à 900.

4. Agent pour utilisation selon la revendication 2, dans lequel le polymère d'acide 3-oxygermylpropionique a les caractéristiques suivantes dans un spectre de diffraction des rayons X sur poudre :
un grand pic de diffraction : autour de 6,5°,
des pics de diffraction relativement grands : autour de 11,6°, 13,8°, 18,4°, 21,2°, et 22,4°, respectivement.

5. Agent pour utilisation selon la revendication 2, dans lequel le polymère d'acide 3-oxygermylpropionique a les caractéristiques d'absorption suivantes dans un spectre IR :
des grandes bandes d'absorption : autour de 800 cm⁻¹, 900 cm⁻¹, et 1700 cm⁻¹, respectivement,
des bandes d'absorption relativement grandes : autour de 560 cm⁻¹, 705 cm⁻¹, 760 cm⁻¹, 780 cm⁻¹, 1250 cm⁻¹, 1350 cm⁻¹, et 1400 cm⁻¹, respectivement, la bande d'absorption autour de 1400 cm⁻¹ étant un doublet.

6. Agent pour utilisation selon la revendication 2, dans lequel le polymère d'acide 3-oxygermylpropionique a les caractéristiques suivantes sur une courbe de calorimétrie différentielle à balayage (DSC) :
point d'initiation du pic : autour de 237 °C,
sommet du pic : autour de 256 °C,
point de fin du pic : autour de 276 °C,
quantité de chaleur ΔH = environ 59 mcal/mg.

7. Agent pour utilisation selon la revendication 2, dans lequel le polymère d'acide 3-oxygermylpropionique est un composé qui a :
comme formule brute : (C₃H₅GeO_{3,5})ₙ,
comme degré de polymérisation moyen en poids : n = 548 ± 337, et
comme masse moléculaire moyenne en poids : valeur moyenne ± erreur type = 9,29 x 10⁴ ± 5,72 x 10⁴.
